# EUROPEAN PATENT APPLICATION

(11) **EP 1 611 894 A1**
(43) Date of publication of application: **04.01.2006**
(21) Application number: 04723952.0
(22) Date of filing: 29.03.2004
(51) Int. Cl.: A61K 31/7008, A61P 31/00

(54) **USE OF N-ACETYL-D-AMINOGLYCOSAMINE IN TREATMENT OF NON-SPECIFIC INFLAMMATIONS RELATED TO PHYSICAL OR CHEMICAL FACTORS**

(30) Priority: 27.03.2003 CN 03108278
(71) Applicant: Third Military Medical University Chinese People's Liberation Army P.R. of China, Chongqing 400038 (CN); Beijing Sino-Hongkong Dafu Science & Technology of Biowave Co., Ltd., Beijing 101200 (CN)
(72) Inventor: XU, Qiwang, Shapingba District Chongqing 400038 (CN); LIU, Junkang, Shapingba Dis trict Chongqing 400038 (CN); YUAN, Zetao, Chongqing 400038 (CN)
(74) Representative: Giambrocono, Alfonso
(86) International application number: PCT/CN2004/000280
(87) International publication number: WO 2004/084915

(57) **Abstract**

The present invention relates to a use of N-acetyl-D-glucosamine in the manufacture of a medicament for treating non-specific inflammations caused by physical/chemical factors and controlling symptoms thereof. A preparation comprising N-acetyl-D-glucosamine as main active component for treating non-specific inflammations caused by physical/chemical factors exhibits merits of significant therapeutical effects and simple manufacture and has no side effect.

## Description

### Technical field

The present invention relates to the use of N-acetyl-D-glucosamine or pharmaceutically acceptable salts thereof for in treating non-specific inflammations caused by physical or chemical factors and controlling symptoms thereof, and the use of N-acetyl-D-glucosamine or pharmaceutically acceptable salts thereof in the manufacture of a medicament for treating non-specific inflammations caused by physical or chemical factors and for controlling symptoms thereof.

### Background Art

Many factors cause non-specific inflammations, wherein common physical factors relate to scorching hot, insolation, crush injury, cold injury, blunt tearing, etc.; and common chemical factors include strong acid burn, strong base burn or lime burn, etc. Under the action of said factors, non-specific inflammations occur in organisms, i.e., local congestion, edema, increase of secretory products and exudates, pain, even skin destruction and necrosis appear, while specific infective pathogen cannot be found in the region, so that it is called "non-specific inflammations". The mechanism of non-specific inflammation lies in that vital movements of organism in different levels are concordant under normal situation, while components such as structural proteins, etc. of histiocyte are directly destroyed when physical or chemical factors occur rapidly, the cellular structure is destroyed or the environment of cells is deteriorated, the intermediate products, such as lactic acid, pyruvic acid, etc., of tri-carboxylic acid cycle accumulate in the local environment due to the continuation of original cell metabolism, and the stability of lysosomal membrane decreases due to the lack of energy supply and the accumulation of H⁺, which result in a series of non-specific inflammations in cells. At present, non-specific inflammations still cannot be effectively treated in China and foreign countries. Supporting therapies, such as local rinsing, wet compress and transfusion, are commonly used in practical, which mainly are to oppose secondary infections, belong to conservative maintenance therapy, and cannot control and prevent the further extension of injury. Thus, the development of new therapeutical drugs is still in need.

In the research of "bio-waves" theory, the present inventor has set up a organism wave-growth model. Through researching, it is known that this wave is of its intrinsic regulation mechanism: some chemical substances are able to participate the regulation in the bio-wave process, so as to transform an abnormal periodic slow wave into a normal physiological chaotic quick wave, and this kind of substances are known as wave-promoting factors. Through separating, purifying and identifying, it is determined that one of the wave-promoting factors is N-acetyl-D-glucosamine. N-acetyl-D-glucosamine has characteristics of general chiral drugs, but is different from common chiral drugs, i.e., it exhibits an autovibration of interconversion of its two enantiomers. This diphase variation results in a random high-affinity interaction between N-acetyl-D-glucosamine and components in vivo, such as cells, subcellular biomacromolecules, etc., so that the configuration and conformation of these components are stabilized, and the further extension of variation caused by the original harmful factors is inhibited.

N-acetyl-D-glucosamine is a chemical reagent. From the 1990's, it is continually used to treat diseases such as pericementitis (WO9102530A1), microbiological infection (WO9718790A3), intestinal inflammation (WO9953929A1), cornea disease (JP10287570A2), hypertrophy of the prostate (US05116615), and so on. It is also applied in cosmetology (JP59013708A2), shampoo preparation (JP2011505A2), and the like, but it has not been used in the manufacture of a medicament for treating non-specific inflammations caused by physical or chemical factors and controlling symptoms thereof.

The inventor of the present invention surprisingly finds that N-acetyl-D-glucosamine and pharmaceutically acceptable salts rapidly and effectively treat non-specific inflammations caused by physical and/or chemical factors and control symptoms thereof, and can prevent the further extension of injury, thereby the present invention is carried out.

### Contents of the invention

One object of the present invention is to provide a use of N-acetyl-D-glucosamine or pharmaceutically acceptable salts thereof in the treatment of non-specific inflammations caused by physical and/or chemical factors.

Another object of the present invention is to provide a use of N-acetyl-D-glucosamine or pharmaceutically acceptable salts thereof in the manufacture of a medicament for the treatment of non-specific inflammations caused by physical and/or chemical factors.

Another object of the present invention is to provide a method for treating non-specific inflammations caused by physical and/or chemical factors, comprising administering a patient a pharmaceutical composition comprising an effective amount of N-acetyl-D-glucosamine or pharmaceutically acceptable salts thereof.

The above objects of the present invention are achieved by: using N-acetyl-D-glucosamine or pharmaceutically acceptable salts thereof as active component in combination with optional pharmaceutically acceptable excipients or carriers in the art to manufacture a preparation in aqueous form, vinum form, ointment form, powder form or injectable form for treating and controlling non-specific inflammations caused by physical and/or chemical factors.

The said N-acetyl-D-glucosamine is a compound having a molecular formula of C₈H₁₅NO₆ and a structure formula (I).

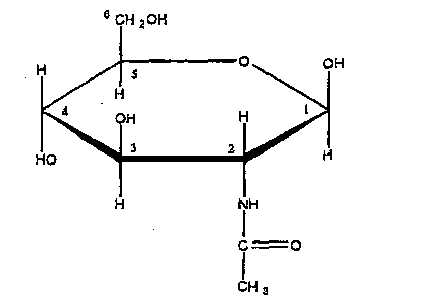

The examples of pharmaceutical acceptable salts of N-acetyl-D-glucosamine that can be used in the present invention include, but are not limited to the salts formed with inorganic acids, such as hydrochloride, hydrobromide, borate, phosphate, sulfate, hydrosulfate and hydrophosphate, and the salts formed with organic acids, such as citrate, benzoate, ascorbate, methylsulfate, picrate, fumarate, maleate, malonate, succinate, tartrate, mesylate, and glucose-1-phosphate.

In the pharmaceutical composition of the present invention, the content of N-acetyl-D-glucosamine or pharmaceutically acceptable salts thereof is generally 0.1-10% by weight.

According to the above statements, N-acetyl-D-glucosamine or pharmaceutically acceptable salts thereof can combine with various pharmaceutically accept excipients or carriers in the art to manufacture a preparation in aqueous form, vinum form, ointment form, powder form or injectable form. The pharmaceutical composition of the present invention can be administered via various routes, such as injection, oral administration, local coating, local rinsing, or a combination thereof.

It is conventional techniques in the art to select kinds and amounts of excipients or carriers according to the dose form of the pharmaceutical composition. For example, N-acetyl-D-glucosamine or pharmaceutically acceptable salts thereof can be dispersed in water for injection, oily solvent, such as fat oil, etc., to form a solution or suspension for parenteral administration, or can be diluted with a diluent, such as 0.9% physiological saline for injection, sterilized water for injection, or 0.5% lidocaine hydrochloride, and further diluted with 5% glucose solution or injectable 0.9% physiological saline for intramuscular injection or intravenous injection, or can be combined with Vaseline as excipient for manufacturing an ointment for external application.

The pharmaceutical composition comprising N-acetyl-D-glucosamine or pharmaceutically acceptable salts thereof in the present invention can be administered in a manner of single dose per day or multidoses per day. The dose of said pharmaceutical composition depends on patient's age, condition, symptom, and administration manner. In general, as to an adult patient having a bodyweight of 75 kg, the dose of said pharmaceutical composition is 1-100000 mg per day, preferably 10-10000 mg per day, based on active component.

Although the present invention is not restricted by any specific theory, it is believed that N-acetyl-D-glucosamine or pharmaceutically acceptable salts thereof can regulate bio-wave, promote cell redistribution and stabilize lysosomal membrane under disadvantageous environments, thereby treating and controlling non-specific inflammations and preventing the further extension of injury. This compound is substantively nontoxic and can avoid many side-effects caused by drugs, such as hormones and so on that are used for the treatment of non-specific inflammations.

### Embodiments for carrying out the invention

The beneficial effects of the present invention are further demonstrated by the following examples, but it shall be understand that these examples are merely to illustrate the present invention, rather than to restrict the scope of the present invention in any aspect.

### Example 1. Promoting wave test of the compound of formula (I)

### 1. Experimental materials and method:

### 1.1 Sample: pure compound of formula (I)

### 1.2 Experimental materials:

Strain: *Proteus Mirabilis* that meets the following biochemical reaction characteristics: dynamics (+), urease (+), lactose (-), glucose (+), H₂S (-), phenylalanine deaminase (+).
Culture medium: modified LB culture medium (components: 1% tryptones, 0.5% yeast extract, 1% sodium chloride, 0.1% glucose, 0.002% TTC, and pH = 7.2 to 7.4).

### 1.3 Experimental method:

Control sample: the *Proteus Mirabilis* were inoculated at the center of LB plate, and incubated at 37°C for 9 hours;
Test sample: the compound of formula (I) with a final concentration of 0.5% was added to the LB plate, then the *Proteus Mirabilis* were inoculated by the same method, and cultured at 37°C for 9 hours.

### 2. Experimental results and evaluation:

The control sample exhibited concentric rings with an interval of 3 hours, which extended outward continually. The test sample showed not only concentric rings with an interval of 3 hours, but also many fine waves on each ring in comparison with the control sample.
The experiment adopts a bio-wave model to research the promoting wave function of the compound of formula (I). The results showed that the compound of formula (I) was not only able to cause bacterial cell to reveal a normal bio-wave characteristic, but also cause the wave reveal finer wave mode. These indicated that the compound of formula (I) has a function of promoting bio-waves. This wave-promoting function may explain the mechanism of using N-acetyl-D-glucosamine or pharmaceutically acceptable salts thereof for treating non-specific inflammation caused by physical and/or chemical factors and controlling symptoms thereof.

### Example 2. Toxicological test of the compound of formula (I)

The toxicological test of the compound of formula (I) includes:
1. Acute toxicity test: including tests of oral administration, intravenous injection administration, and maximum limit amount for administration;
2. Ames test;
3. Micronucleus test of mouse bone marrow cell;
4. Abnormality test of mouse sperm;
5. Aberration test of mouse testis chromosome;
6. Chronic lethal test;
7. Sub-chronic toxicity (feed for 90 days) test;
8. Traditional deformity-inducing test.

The results of these tests showed that in the acute toxicity test of the compound of formula (I), the acute toxicosis reaction had not appeared when the dosage more than 2 g/kg was taken; in the long-period toxicity test, the maximum dosage had reached up to 1 g/kg, and after the treatment and observation for four weeks, there was no intoxication reaction yet; and in the reproduction test, the mice were feed with a routine dosage of 7 mg/kg for 3 generations, it had been proved that the compound of formula (I) had no influence on the pregnancy, birth, nurse and the growth of baby mice, so that the compound of formula (I) is a substance without toxicity.

### Example 3. Animal tests

### 1. Burn test

20 Wistar rats were randomly divided into two groups, i.e., control group and test group. The rats of both two groups were made as burn models by dressing gauze with hot distilled water. The rats of the control group were not treated, while the rats of the test group were locally treated by applying a solid powder of the compound of formula (I). After 3 days, 8 rats of the test group formed scab, while only 2 rats of the control group formed scab wherein liquor puris still accumulated below the scab. The two groups were significantly different in burn reaction.
When N-acetyl-D-glucosamine was replaced with N-acetyl-D-glucosamine hydrochloride in the test group, 6 rats of the test group formed scab and exhibited well-repaired wound surface. The test group was significantly different from the control group.

### 2. Blunt tearing tests of animals

30 Kunming mice were randomly divided into two groups, i.e., 10 mice in control group and 20 mice in test group. The mice of both two groups were treated by a blunt tearing method, wherein a non-specific injury occurred in blunt torn thigh muscle tissue of hind limb of mice. The injured limb could not freely flex and stretch and arbitrarily move, and exhibited local swelling and pain (could not be touched). 10 mice of control group were normally fed, while the mice of test group were administered with an aqueous solution of N-acetyl-D-glucosamine having a concentration of 5% by weight in a dosage of 1 ml, wherein 10 mice were administered by intra-peritoneal injection, another 10 mice were administered by intramuscular injection. After 7 days of administration, the rats of two groups exhibited different reactions. The pathological changes of the mice of control group continuously developed or maintained on the original basis, wherein 4 mice suffered paralysis of the injured limb, and another 4 mice had flex and stretch dysfunction. The mice of test group could free-run, except one mouse in intra-peritoneal injection group suffered paralysis. The two groups were significantly different.
When N-acetyl-D-glucosamine was replaced with N-acetyl-D-glucosamine sulfate in the tests (dosage and administration manner were same as above), similar results were obtained. In the test group, 2 mice had flex and stretch dysfunction, and 2 mice suffered paralysis of the injured limb. The test group was significantly different from the control group.

### Example 4. Test for treatment of burn

In a collaborate project of the burn department of Third Military Medical University, a sterilized N-acetyl-D-glucosamine powder in a dose dependant upon wound surface area was applied to the wound surface of 10 burned voluntary patients for consecutive 3 days. From the 4^{th} day, cambium continuously grew around the wound and extended toward the center of the wound at a rate of 1-2 mm per day, and in the meantime, edema was alleviated, blood vessel shrank, and secretion was reduced.

### Example 5. Test for treatment of surgical trauma

8 Patients with surgical trauma, and most of them were facial and forearm abrasion. An ointment with a concentration of 1g/10g was prepared by mixing N-acetyl-D-glucosamine and Vaseline, and was painted on wounded parts in a dose dependent upon the area of wound surface for consecutive 3 days. Facial abrasion was rapidly healed and no scar was found on face.

### Example 6. Test for treatment cold injury

20 Patients with different levels of cold injury were divided into a test group and a control group. The patients in the test group were locally treated with a 10wt% N-acetyl-D-glucosamine aqueous solution in a dose from 1 ml to 10 ml per day according to symptoms and individual conditions. The test group exhibited early recovery, and then inflammation and edema were eliminated. 10 Patients in the control group exhibited continuous and obvious inflammatory symptoms of rubor, edema and pain.

## Claims

1. A use of N-acetyl-D-glucosamine or pharmaceutically acceptable salts thereof in the manufacture of a medicament for treating non-specific inflammations caused by physical/chemical factors.

2. A use according to claim 1, wherein said medicament is of aqueous form, vinum form, ointment form, powder form or injectable form.

3. A use according to 1 or 2, wherein the N-acetyl-D-glucosamine in said medicament has a concentration of 0.1-10% by weight.

4. A use according to 1 or 2, wherein the dose of said medicament is 1-100000 mg/day/75kg body weight, based on the active component.

5. A method for treating non-specific inflammations caused by physical/chemical factors, comprising administering a patient a pharmaceutical composition comprising an effective amount of N-acetyl-D-glucosamine or pharmaceutically acceptable salts thereof.

6. A method according to claim 5, wherein said pharmaceutical composition is of aqueous form, vinum form, ointment form, powder form or injectable form.

7. A method according to 5 or 6, wherein the dose of said pharmaceutical composition is 1-100000 mg/day/75 kg bodyweight, based on the active component.

8. A use of N-acetyl-D-glucosamine or pharmaceutically acceptable salts thereof in treating non-specific inflammations caused by physical/chemical factors.
